(19) Europäisches Patentamt
European Patent Office
Office européen des brevets

(11) Veröffentlichungsnummer: **0 299 329 B1**

(12) **EUROPÄISCHE PATENTSCHRIFT**

(45) Veröffentlichungstag der Patentschrift: **29.09.93**

(51) Int. Cl.[5]: **C07C 323/62**

(21) Anmeldenummer: **88110689.2**

(22) Anmeldetag: **05.07.88**

# (54) Verfahren zur Herstellung von 2-Arylthiobenzoesäuren.

(30) Priorität: **11.07.87 DE 3723079**

(43) Veröffentlichungstag der Anmeldung:
**18.01.89 Patentblatt 89/03**

(45) Bekanntmachung des Hinweises auf die Patenterteilung:
**29.09.93 Patentblatt 93/39**

(84) Benannte Vertragsstaaten:
**CH DE FR GB IT LI**

(56) Entgegenhaltungen:
**EP-A- 0 272 742**
**US-A- 4 094 900**

**CHEMICAL ABSTRACTS, Band 108, 1. - 15. Februar 1988, Seite 553, Zusammenfassung Nr. 36939a, Columbus, Ohio, US; O.G. KULINKOVICH et al.: "Reaction of alpha-bromocyclopropyl phenyl ketones with lithium thiophenolate via a concealed nucleophilic substitution mechanism at the halogen atom"**

(73) Patentinhaber: **CASSELLA Aktiengesellschaft**
**Hanauer Landstrasse 526**
**D-60386 Frankfurt(DE)**

(72) Erfinder: **Bauer, Wolfgang, Dr. Dipl.-Chem.**
**Masurenstrasse 6**
**D-6457 Maintal 3(DE)**
Erfinder: **Langer, Manfred, Dr. Dipl.-Chem.**
**Birsteiner Strasse 47**
**D-6000 Frankfurt am Main 61(DE)**
Erfinder: **Reh, Kuno, Dr. Dipl.-Chem.**
**Lauterbacher Strasse 14**
**D-6000 Frankfurt/Main 61(DE)**

(74) Vertreter: **Urbach, Hans-Georg, Dr. et al**
**Hanauer Landstrasse 526**
**D-60386 Frankfurt (DE)**

EP 0 299 329 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein neues Verfahren zur Herstellung von 2-Arylthiobenzoesäuren der Formel I

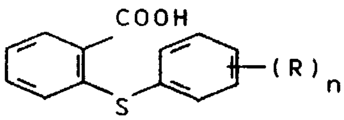

(I)

worin

R Alkyl mit 1 bis 4 Kohlenstoffatomen, Alkoxy mit 1 bis 4 Kohlenstoffatomen, Trifluormethyl, Wasserstoff, Fluor, Chlor oder Nitro und

n 1, 2, 3 oder 4 bedeutet, wobei die Reste R gleich oder verschieden sein können, durch Umsetzung von Lithium-2-chlorbenzoat der Formel II

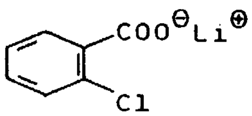

(II)

mit Lithium-thiophenolat der Formel III

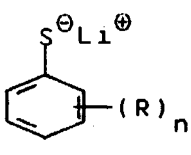

(III)

worin R und n die oben angegebenen Bedeutungen haben.

2-Arylthiobenzoesäuren der Formel I sind wertvolle Zwischenprodukte zur Herstellung von Thioxanthenen, die z.B. als Photoinitiatoren für UV-härtende Lacke (M.J. Davis et al., J. Oil Col. Chem. Assoc. 61. 256 (1978)) oder als Ausgangsprodukte für stark wirksame Neuroleptika (H.J. Roth u. A. Kleemann, Pharmazeutische Chemie I, Arzneistoffsynthese, G. Thieme Verlag Stuttgart, 1982, S. 284-286) Verwendung finden.

Es sind bereits verschiedene Verfahren zur Herstellung von 2-Arylthiobenzoesäuren bekannt, nach denen 2-Halogenbenzoesäuren mit Thiophenolen umgesetzt werden können.

Beispielsweise reagiert nach der in der US-Patentschrift 4094900 angegebenen Verfahrensweise Natrium-2-chlorbenzoat mit Natrium-4-chlorthiophenolat in Gegenwart äquimolarer Mengen Natriumjodid bei 170 °C (7 Stunden) zu 2-(4-Chlorphenylthio)-benzoesäure. Der Nachteil dieser Methode liegt in der Verwendung äquimolarer Mengen Natriumjodid, denn durch die hohen Kosten für Natriumjodid ist dieses Verfahren unwirtschaftlich. Zusätzlich tritt eine hohe Abwasserbelastung durch nicht wiedergewonnenes Natriumjodid auf.

Ein weiteres literaturbekanntes Verfahren ist durch die Ullmann-Reaktion von 2-Jod-benzoesäure mit Thiophenolen in Kalilauge und Gegenwart von Kupfer (J.O. Jilek et al., Collect. Czechoslov. Chem. Commun. 33, 1831 (1968), Französ. Patentschriften M. 6165, 5964) oder von 2-Chlorbenzoesäure mit Thiophenolen in Nitrobenzol in Gegenwart von Kaliumcarbonat und Kupferjodid und Kupferbronze als Katalysatoren (J.D. Brindle et al., Can. J. Chem. 61, 1869 (1983)) gegeben. Diese Reaktionen weisen als Nachteile die Verwendung von teuren Ausgangsmaterialien wie 2-Jod-benzoesäure sowie den Einsatz von Kupfer bzw. Kupferjodid auf. Die Verwendung von Kupfer als Katalysator zieht außerdem die Bildung von Nebenprodukten und damit schlechtere Ausbeuten an 2-Arylthiobenzoesäuren nach sich.

Der vorliegenden Erfindung lag die Aufgabe zugrunde, ein Verfahren zur Herstellung von 2-Arylthiobenzoesäuren zur Verfügung zu stellen, das die wirtschaftlichen und ökologischen Nachteile der angegebenen literaturbekannten Verfahren nicht aufweist.

Die Lösung dieser Aufgabe gelingt überraschenderweise dadurch, daß man Lithium-2-chlorbenzoat der Formel II mit Lithium-thiophenolat der Formel III zur Reaktion bringt.

Dabei kann auf den Einsatz von teuren Katalysatoren wie äquimolaren Mengen Natriumjodid, Kupfer, Kupferjodid oder auf den Einsatz teurer Edukte wie 2-Jod-benzoesäure verzichtet werden.

Die Reaktion wird in der Regel bei Temperaturen von 140 bis 220 °C, vorzugsweise bei 170 bis 200 °C, ausgeführt.

Üblicherweise werden pro mol Thiophenolat 1 bis 1,5 mol, vorzugsweise 1,1 bis 1,3 mol, Chlorbenzoat eingesetzt.

Das Verfahren kann entweder ohne Verwendung eines Lösungsmittels in der Schmelze oder in aprotischen Lösungsmitteln bei Normaldruck oder unter Druck in einem Autoklaven durchgeführt werden.

Geeignete Lösungsmittel sind beispielsweise: Toluol, o-, m-, p-Xylol, Mesitylen, Chlorbenzol, o-Chlortoluol, o-Dichlorbenzol, Tetralin, Solventnaphtha, Dibutylether, Diphenylether, Dimethylformamid, Dimethylacetamid, N-Methylpyrrolidon, N,N-Dimethylpropylenharnstoff, Dimethylsulfoxid, Dimethylanilin.

Das erfindungsgemäße Verfahren läßt sich in vorteilhafter Weise durchführen, indem man 2-Chlorbenzoesäure der Formel IV

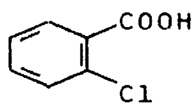

(IV)

und ein Thiophenol der Formel V,

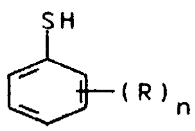

(V)

worin R und n die bereits genannten Bedeutungen haben, gegebenenfalls in einem geeigneten Lösungsmittel mischt, mit molaren Mengen Lithiumhydroxid und/oder Lithiumcarbonat und/oder Lithiumphosphat in die entsprechenden Lithiumsalze (Formel II bzw. III) überführt und das Reaktionswasser bei Normaldruck oder im Vakuum durch Destillation entfernt und anschließend auf die gewünschte Reaktionstemperatur erhitzt. Die Reaktion ist normalerweise in 2 bis 8 Stunden beendet.

Nach Vervollständigung der Reaktion wird das entstandene Lithium-2-arylthio-benzoat der Formel VI

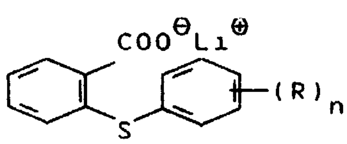

(VI)

in Wasser aufgenommen und mit einer Mineralsäure in die freie Säure (Formel I) übergeführt. Geeignete Mineralsäuren sind zum Beispiel Salzsäure, Schwefelsäure und Phosphorsäure.

Bei Verwendung von mit Wasser nicht oder schwer mischbaren Lösungsmitteln wird die wäßrige, die Verbindung der Formel VI enthaltende Phase, von der organischen Phase abgetrennt, wobei die organische Phase ohne weitere Reinigungsoperation für Folgeansätze wieder eingesetzt werden kann.

Setzt man aprotische, mit Wasser mischbare Lösungsmittel ein, wird dieses vor dem Wasserzusatz durch Destillation entfernt.

Die in den allgemeinen Formeln I, III und V genannten Reste R können gleich oder verschieden sein. Sie können die 2-, 3-, 4- und 5-Stellung des aromatischen Ringes substituieren. Für n = 1 sind die 2-, 3- und 4-Stellungen bevorzugt. Ganz besonders bevorzugt ist die 4-Stellung.

Für n = 2 können die Substituenten R in 2,3-, 2,4-, 2,5-, 3,4-, 3,5- und 4,5-Stellung stehen. Für n = 3 sind ebenfalls alle denkbaren Kombinationen möglich,bevorzugt ist die 2,4,5-Substitution.

Als Thiophenole der Formel V können nach dem erfindungsgemäßen Verfahren beispielsweise eingesetzt werden: Thiophenol, 4-Fluor-thiophenol, 3-Fluor-thiophenol, 4-Chlor-thiophenol, 2-Chlor-thiophenol, 2,5-Dichlor-thiophenol, 4-Chlor-2,5-dimethyl-thiophenol, 4-Methyl-thiophenol, 4-Isopropyl-thiophenol, 3-Trifluormethyl-thiophenol, 4-Nitro-thiophenol, 4-Methoxy-thiophenol.

Beispiel 1

2-(4-Chlorphenylthio)-benzoesäure

Eine Mischung von 144,6 g 4-Chlor-thiophenol und 172,2 g 2-Chlor-benzoesäure in 450 g Tetralin wird mit 96,4 g Lithiumhydroxid-monohydrat versetzt und am Wasserabscheider zur Entfernung von ca. 54 ml Reaktionswasser auf 185-190 °C geheizt.

Anschließend rührt man 8 Stunden bei 185-190 °C nach, kühlt das Reaktionsgemisch auf 115 °C und fügt 500 ml Wasser zu.

Die wäßrige Produktphase wird abgetrennt, mit 1,5 l Wasser verdünnt und durch Zusatz von 125 g Salzsäure 30%ig auf pH 2 gestellt.

Die ausgefallene 2-(4-Chlorphenylthio)-benzoesäure wird durch Filtration isoliert, mit Wasser gewaschen und getrocknet.

Ausbeute: 290,2 g (95% d.Theorie, bezogen auf 4-Chlorthiophenol)
Reinheitsgrad: 96% (HPLC)
Schmelzpunkt: 230-235 °C

Das Produkt eignet sich hervorragend zur Herstellung von reinem 2-Chlor-thioxanthon durch saure Cyclisierung.

Vergleichsbeispiele

Verfährt man nach den Angaben des Beispiels 1, setzt jedoch statt 96,4 g Lithiumhydroxid-monohydrat 92 g Natriumhydroxid bzw. 123 g Kaliumhydoxid ein, erhält man folgende Ergebnisse:

| eingesetztes Alkalihydroxid | Ausbeute an I, bez. auf 4-Chlor-thiophenol (% d.Th.) | Reingehalt an I der erhaltenen Rohware | Schmelzp. (°C) |
|---|---|---|---|
| NaOH | 54 | 65 % | 170-198 |
| KOH | 52 | 59 % | 190-198 |

In der folgenden Tabelle sind weitere 2-Arylthiobenzoesäuren aufgeführt, die nach den Angaben des Beispiels 1 durch Umsetzung von Lithium-2-chlor-benzoat mit Lithium-thiophenolaten hergestellt werden können.

In der Tabelle ist angegeben:
in Spalte 1: das eingesetzte Thiophenol der Formel V
in Spalte 2: die erhaltene 2-Arylthiobenzoesäure der Formel I
in Spalte 3: die Ausbeute an I, bezogen auf das eingesetzte Thiophenol
in Spalte 4: der Schmelzpunkt der erhaltenen 2-Arylthiobenzoesäure

2-Arylthiobenzoesäuren aus Lithium-2-chlor-benzoat und Lithium-thiophenolaten

| Beispiel | Thiophenol (V) | 2-Arylthiobenzoesäure (I) | Ausbeute (% d.Th.) | Schmelzpunkt (°C) |
|---|---|---|---|---|
| 2 | 4-Methyl-thiophenol | 2-Phenylthio-benzoesäure | 85 | 164 - 167 |
| 3 | 4-Fluor-thiophenol | 2-(4-Fluorphenylthio)-benzoesäure | 86 | 172 - 170 |
| 4 | 4-Methylthiophenol | 2-(4-Methylphenylthio-benzoesäure | 89 | 210 - 215 |
| 5 | 4-Isopropyl-thio phenol | 2-(4-Isopropylphenylthio)-benzoesäure | 85 | 148 - 152 |
| 6 | 2-Chlor-thiophenol | 2-(2-Chlorphenylthio)-benzoesäure | 80 | 169 - 172 |
| 7 | 4-Methoxy-thio-phenol | 2-(4-Methoxyphenylthio)-benzoesäure | 83 | 225 - 229 |
| 8 | 4-Chlor-2,5-di-methyl-thiophenol | 2-(4-Chlor-2,5-dimethylphenylthio)-benzoesäure | 84 | 182 - 185 |
| 9 | 4-Nitro-thiophenol | 2-(4-Nitrophenylthio)-benzoesäure | 80 | 221 - 225 |
| 10 | 3-Trifluormethyl-thiophenol | 2-(3-Trifluormethylphenylthio)-benzoesäure | 83 | 149 - 153 |

## Patentansprüche

1. Verfahren zur Herstellung von 2-Arylthiobenzoesäuren der Formel I,

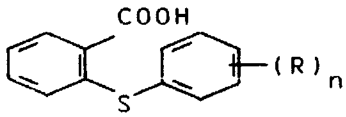

(I)

worin

R Alkyl mit 1 bis 4 Kohlenstoffatomen, Alkoxy mit 1 bis 4 Kohlenstoffatomen, Trifluormethyl, Wasserstoff, Fluor, Chlor oder Nitro und

n 1, 2, 3 oder 4 bedeutet,

wobei die Reste R gleich oder verschieden sein können,

dadurch gekennzeichnet, daß Lithium-2-chlorbenzoat der Formel II

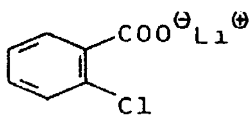

(II)

mit Lithium-thiophenolat der Formel III

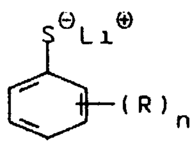

(III)

worin R und n die oben genannten Bedeutungen haben, umgesetzt wird.

2. Verfahren nach Anspruch 1, dadurch gekennzeichnet, daß die Umsetzung bei Temperaturen von 140 bis 220 °C, vorzugsweise bei 170 bis 200 °C ausgeführt wird.

3. Verfahren nach Ansprüchen 1 und 2, dadurch gekennzeichnet, daß pro mol Lithium-thiophenolat der Formel III 1 bis 1,5 mol, vorzugsweise 1,1 bis 1,3 mol, Lithium-2-chlorbenzoat der Formel II eingesetzt werden.

4. Verfahren nach Ansprüchen 1 bis 3, dadurch gekennzeichnet, daß die Reaktion in einem aprotischen Lösungsmittel ausgeführt wird.

5. Verfahren nach Ansprüchen 1 bis 3, dadurch gekennzeichnet, daß die Reaktion lösungsmittelfrei in der Schmelze ausgeführt wird.

## Claims

1. Process for the preparation of 2-arylthiobenzoic acids of the formula I

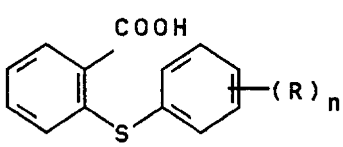

(I)

wherein
R denotes alkyl having 1 to 4 carbon atoms, alkoxy having 1 to 4 carbon atoms, trifluoromethyl, hydrogen, fluorine, chlorine or nitro and
n denotes 1, 2, 3 or 4,
where the R radicals can be identical or different,
characterized in that lithium 2-chlorobenzoate of the formula II

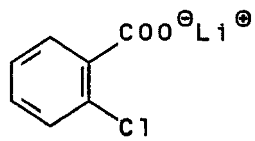

(II)

is reacted with lithium thiophenolate of the formula III

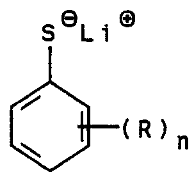

(III)

wherein R and n have the abovementioned meanings.

2. Process according to Claim 1, characterized in that the reaction is carried out at temperatures of 140 to 220 ° C, preferably at 170 to 200 ° C.

3. Process according to Claims 1 and 2, characterized in that 1 to 1.5 moles, preferably 1.1 to 1.3 moles, of lithium 2-chlorobenzoate of the formula II are employed per mole of lithium thiophenolate of the formula III.

4. Process according to Claims 1 to 3, characterized in that the reaction is carried out in an aprotic solvent.

5. Process according to Claims 1 to 3, characterized in that the reaction is carried out in the melt without a solvent.

## Revendications

1. Procédé de préparation d'acides 2-arylthiobenzoïques de formule I ci-dessus

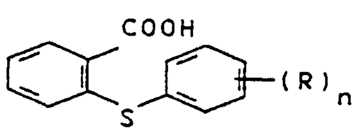

(I)

dans laquelle
R représente un alkyle ou un alcoxy ayant chacun de 1 à 4 atomes de carbone, le groupe trifluorométhyle, un atome d'hydrogène, de fluor ou de chlore ou encore le groupe nitro et
n le nombre 1, 2, 3 ou 4,
les radicaux R pouvant être identiques ou différents les uns des autres,
procédé caractérisé en ce que l'on fait réagir le 2-chlorobenzoate de lithium de formule II

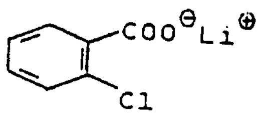

(II)

avec un thiophénolate de lithium de formule III

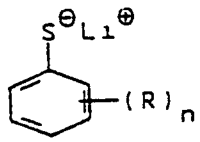

(III)

R et n ayant les mêmes significations que pour la formule I.

2. Procédé selon la revendication 1, caractérisé en ce que l'on effectue la réaction à des températures de 140 à 220 ° C, de préférence de 170 à 200 ° C.

3. Procédé selon les revendications 1 et 2, caractérisé en ce que l'on opère avec de 1 à 1,5 mol du 2-chlorobenzoate de lithium de formule II, de préférence avec 1,1 à 1,3 mol, par mol du thiophénolate de lithium de formule III.

4. Procédé selon les revendications 1 à 3, caractérisé en ce que l'on effectue la réaction dans un solvant aprotique.

5. Procédé selon les revendications 1 à 3, caractérisé en ce que l'on effectue la réaction sans solvant, dans la masse fondue des réactifs.